# EUROPÄISCHE PATENTSCHRIFT

(11) **EP 2 374 461 B1**
(45) Veröffentlichungstag und Bekanntmachung des Hinweises auf die Patenterteilung: **26.10.2016**
(21) Anmeldenummer: 11001816.5
(22) Anmeldetag: 04.03.2011
(51) Int. Cl.: A61K 31/5415, A61P 31/12, A61P 31/20, A61P 31/22, A61K 9/06, A61K 9/00

(54) **Piroxicam zur prohylaktischen und therapeutischen Behandlung von Herpes-Infektionen**
Piroxicam for prophylactic and therapeutic treatment of herpes infections
Piroxicam pour traitement prophylactique et thérapeutique d'infections de type Herpès

(30) Priorität: 08.04.2010 DE 102010014290
(43) Veröffentlichungstag der Anmeldung: 12.10.2011
(73) Patentinhaber: HOV GmbH, 30169 Hannover (DE); Slomianny, Jan, 51588 Nümbrecht (DE)
(72) Erfinder: Slomianny, Jan, 51588 Nümbrecht (DE)
(74) Vertreter: Thiel, Christian

(56) Entgegenhaltungen:
- WO-A1-2004/060360
- DATABASE BIOSIS [Online] BIOSCIENCES INFORMATION SERVICE, PHILADELPHIA, PA, US; 1984, OGINO A ET AL: "THERAPEUTIC EFFECTS OF FELDENE PIROXICAM ON HERPES ZOSTER", XP002637068, Database accession no. PREV198579102385 & ACTA DERMATOLOGICA (KYOTO), Bd. 79, Nr. 4, 1984, Seiten 315-320, ISSN: 0065-1176

## Beschreibung

Die Erfindung betrifft ein Mittel zur Anwendung bei der prophylaktischen und therapeutischen Behandlung von Herpes-Infektionen, insbesondere mit Herpes simplex.

Die Behandlung von Virusinfektionen in Mensch und Tier stellt nach wie vor eine Herausforderung dar, da die Zahl der Wirkstoffe begrenzt ist. Dies gilt auch für die Viren der Herpesfamilie.

Die Familie der Herpesviren (Herpesviridae) umfasst eine große Anzahl von Viren mit doppelsträngiger DNA. Weit verbreitet sind die Herpes simplex-Viren vom Typ HSV 1 und HSV 2 sowie das Varicella-Zoster-Virus VZV. Alle verursachen schmerzhafte Infektionen, die sich in oberflächlichen Entzündungen äußern. Herpesviren überdauern lange Zeit im menschlichen Körper, so dass es immer wieder zu Ausbrüchen der Krankheit mit zum Teil gravierenden Krankheitserscheinungen kommt.

Herpes simplex-Infektionen treten vor allem im Mundbereich auf und äußern sich in Blasen und Läsionen an den Schleimhäuten und Lippen. Varicella-Zoster-Infektionen äußern sich in der Kindheit zumeist in Form von Windpocken, beim erwachsenen Menschen als Gürtelrose. Letztere führt zu einem schmerzhaften Exanthem im Bereich eines Spinalnervs, etwa im Bereich der Lenden, des Brustkorbs, aber auch im Gesicht. Zoster-Infektionen gehen mit Fieber, Appetitlosigkeit, Gliederschmerzen und Schmerzen im Bereich des Exanthems einher.

Gegen Herpesviren wurden eine Reihe von Mitteln entwickelt, die aber im Wesentlichen alle nur geeignet sind, die Symptome zu lindern, dagegen aber nur einen geringen Einfluss auf den Verlauf der Krankheit nehmen. Zumeist kommt es nur zu einer geringfügigen Verkürzung der Krankheitsdauer. Ein viel eingesetzter Wirkstoff ist Aciclovir.

Grundsätzlich besteht ein Bedarf an Mitteln, mit denen Virusinfektionen, insbesondere auch Herpes-Infektionen wirksam entgegengewirkt werden kann.

Aus der EP 1 457 202 A2 ist die Verwendung von sogenannten nicht-steroiden entzündungshemmenden Mitteln (NSAIDs) zur Behandlung von Herpes-Infektionen beschrieben. Obwohl in der Anmeldung eine große Anzahl von NSAIDs genannt ist, wird eine Wirksamkeit nur für zwei Vertreter dieser Gruppe, Diclofenac und Ketorolac beschrieben und nur für Diclofenac anhand belastbarer Daten belegt. Demnach ist Diclofenac in topischer Anwendung geeignet, den Krankheitsverlauf zu mildern; im Schnitt wird eine Abheilung der Läsionen nach fünf Tagen erreicht. Dies ist zwar eine Verkürzung der normalen Infektionsdauer, die bis zu 10 Tage dauert, jedoch für den Patienten immer noch unbefriedigend.

Aus A. Ogino et al., "Therapeutic Effects of Feldene Piroxicam on Herpes Zoster", Acta Dermatologica (Kyoto) 79, 4 (1984), S. 315 - 320, ist die Anwendung von Piroxicam als Analgetikum bei Herpes zoster Infektionen bekannt. Eine viruzide Wirkung wird nicht offenbart.

G.J. Kapadia et al. beschreiben in "Inhibition of Epstein-Barr virus early antigen activation promoted by 12-O-tetradecanoylphorbol-13-acetate by the nonsteroidal anti-inflammatory drugs", Cancer Letters 161 (2000) 221-229, die mögliche Verwendung von NSAIDs, darunter Piroxicam, als präventiven Mitteln bei der chemischen Krebsbehandlung. In einem Modell wurde die Inhibierung der durch TPA ausgelösten Early-Antigen-Aktivierung beim Epstein-Barr-Virus durch insgesamt 36 getestete NSAIDs bestimmt.

Es wurde jetzt überraschend gefunden, dass ein zur Gruppe der NSAIDs gehörender Wirkstoff, Piroxicam, geeignet ist, Virusinfektionen prophylaktisch und therapeutisch zu behandeln. Piroxicam hat einen positiven Einfluss auf eine Reihe von Herpes-Infektionen, etwa solche vom Typ HSV1 und HSV2. Es ist geeignet, insbesondere Herpes labialis zu verhindern bzw. zu einer raschen Abheilung zu bringen. Piroxicam, 4-Hydroxy-2-methyl-N-pyridin-2-yl-2H-1,2-benzothiazin-3-carboxamid-1,1-dioxid, ist ein COX-Hemmer und wird als Antirheumatikum eingesetzt.

Entsprechend betrifft die Erfindung ein Mittel der eingangs genannten Art, das Piroxicam in einer geeigneten Trägersubstanz enthält.

Das erfindungsgemäße Mittel enthält Piroxicam vorzugsweise in einer Menge von 0,1 bis 10 Gew.-%, vorzugsweise von 0,1 bis 5 und besonders bevorzugt in einer Menge von 0,5 bis 5 Gew.-%. Es kann topisch, oral und parenteral verabreicht werden.

Bevorzugt wird das erfindungsgemäße Mittel bei Infektionen im Mundbereich, gemeinhin als Herpes labialis bezeichnet, eingesetzt. Es handelt sich dabei um Infektionen mit dem Herpes simplex-Virus HSV 1 bzw. HSV 2. Eine Wirksamkeit gegen Herpes Zoster VZV ist ebenfalls gegeben. Bei allen diesen Infektionen bilden sich oberflächlich schmerzhafte Exantheme.

Das Mittel wird in der Regel topisch verabreicht, insbesondere in Form einer Creme, Salbe, eines Gels oder einer Tinktur. Diese enthalten übliche Trägersubstanzen, also in der medizinischen Praxis eingeführte Formulierungen für Cremes, Salben, Gele und Tinkturen.

Daneben ist die Verabreichung in Tablettenform, als Pulver, Infusionslösung oder Injektionslösung ebenfalls möglich.

Im Allgemeinen wird das Mittel von den Patienten 1 bis 5 x angewendet, 1 bis 2 x täglich. Von der Mehrzahl der Patienten wurde eine einmalige Anwendung als erfolgreich bezeichnet.

Zahlreiche Patienten, die häufig unter Herpes Simplex-Infektionen leiden, berichteten, dass eine prophylaktische Anwendung in Stresssituationen, die typischerweise zur Bildung der Herpesexantheme führen, die Exanthembildung verhindert.

### Versuchsbericht 1

Das erfindungsgemäße Mittel wurde an 42 Probanden anhand eines auf dem Markt befindlichen Piroxicamgels mit einem Wirkstoffgehalt von 0,5 Gew.-% getestet, mit den nachstehend aufgeführten Ergebnissen:

Von 42 Probanden wandten 26 das Mittel 1 x und 16 Probanden das Mittel bis zu 5 x an.

Von den 42 Probanden stellten 15 eine Besserung in weniger als einem Tag fest (einschließlich der Verhinderung des Ausbruchs der Erkrankung), 21 eine Besserung in ein bis drei Tagen und zwei eine Besserung in vier bis zehn Tagen. Die Verträglichkeit wurde von 41 Probanden als gut und von einem Probanden als weniger gut bezeichnet. Ein Proband gab an, dass das Mittel nicht geholfen habe.

Die Wirksamkeit von Piroxicam ist an und für sich überraschend. Der Wirkstoff, ursprünglich als Antirheumatikum konzipiert und chemisch korrekt als 4-Hydroxy-2-methyl-N(2-pyridyl)-2H-1,2-benzothiazin-3-carboxamid-1,1-dioxid bezeichnet, wird weithin als Schmerzmittel verabreicht. Er ist chemisch von den oben beschriebenen NSAIDs Diclofenac und Ketorolac völlig verschieden, so dass - aufgrund seiner chemischen Struktur - nicht von einer viruziden Wirkung gegenüber Herpesviren und insbesondere gegenüber Herpes simplex/Herpes labialis ausgegangen werden kann. Im Gegensatz zu Diclofenac und Keterolac ist es geeignet die Exanthembildung zu verhindern.

### Versuchsbericht 2

In einem Screening-Test wurde ein Piroxicam-haltiges Gel mit einem Wirkstoffgehalt von 0,4 % an einer mit Herpesviren vom Typ HSV1 infizierten Kulturen nach Standardmethoden getestet. Der log CD₅₀-Wert betrug anfangs 2,50, der Virustiter lag anfangs bei 7,00. Der Virustiter (log₁₀ TCID₅₀/ml) lag nach 1, 5 und 60 min Einwirkzeit bei weniger als 2,50, was einer Reduktion der Viruszahl um mehr als 99,99 % entspricht.

## Patentansprüche

1. Mittel zur Anwendung bei der prophylaktischen und therapeutischen Behandlung von Virusinfektionen mit einem Virus aus der Familie der Herpesviren, **dadurch gekennzeichnet, dass** es in einer Trägersubstanz Piroxicam enthält.

2. Mittel zur Anwendung bei der prophylaktischen und therapeutischen Behandlung von Virusinfektionen mit einem Virus aus der Familie der Herpesviren nach Anspruch 1, **gekennzeichnet durch** einen Gehalt von 0,1 bis 10 Gew.-% Piroxicam.

3. Mittel zur Anwendung bei der prophylaktischen und therapeutischen Behandlung von Virusinfektionen mit einem Virus aus der Familie der Herpesviren nach Anspruch 2, **gekennzeichnet durch** einen Gehalt an Piroxicam von 1 bis 5 Gew.-%.

4. Mittel zur Anwendung bei der prophylaktischen und therapeutischen Behandlung von Virusinfektionen mit einem Virus aus der Familie der Herpesviren nach einem der vorstehenden Ansprüche, **dadurch gekennzeichnet, dass** die Virusinfektion eine Infektion mit Herpes simplex, insbesondere Herpes labialis ist.

5. Mittel zur Anwendung bei der prophylaktischen und therapeutischen Behandlung von Virusinfektionen mit einem Virus aus der Familie der Herpesviren nach einem der vorstehenden Ansprüche zur topischen Verabreichung.

6. Mittel zur Anwendung bei der prophylaktischen und therapeutischen Behandlung von Virusinfektionen mit einem Virus aus der Familie der Herpesviren nach Anspruch 5, in Form einer Creme, Salbe, Tinktur oder eines Gels.

7. Mittel zur Anwendung bei der prophylaktischen und therapeutischen Behandlung von Virusinfektionen mit einem Virus aus der Familie der Herpesviren nach einem der Ansprüche 1 bis 4 zur Infusion oder Injektion.

## Claims

1. Agent for the use in a prophylactic and therapeutic treatment of virus infections caused by a virus of the family of herpesviridae, **characterized in that** said agent contains piroxicam in a carrier substance.

2. Agent for the use in a prophylactic and therapeutic treatment of virus infections caused by a virus of the family of herpesviridae according to claim 1, **characterized by** a content of piroxicam ranging between 0.1 and 10 % w/w.

3. Agent for the use in a prophylactic and therapeutic treatment of virus infections caused by a virus of the family of herpesviridae according to claim 2, **characterized by** a content of piroxicam ranging between 1 and 5 % w/w.

4. Agent for the use in a prophylactic and therapeutic treatment of virus infections caused by a virus of the family of herpesviridae according to any one of the above claims, **characterized in that** the virus infection is an infection caused by herpes simplex, in particular herpes labialis.

5. Agent for the use in a prophylactic and therapeutic treatment of virus infections caused by a virus of the family of herpesviridae according to any one of the above claims for topical administration.

6. Agent for the use in a prophylactic and therapeutic treatment of virus infections caused by a virus of the family of herpesviridae according to claim 5, in the form of a cream, ointment, tincture or gel.

7. Agent for the use in a prophylactic and therapeutic treatment of virus infections caused by a virus of the family of herpesviridae according to any one of the claims 1 to 4 for infusion or injection.

## Revendications

1. Produit pour une utilisation lors du traitement prophylactique et thérapeutique d'infections virales par un virus de la famille des herpès virus, **caractérisé en ce qu'**il contient du piroxicam dans une substance support.

2. Produit pour une utilisation lors du traitement prophylactique et thérapeutique d'infections virales par un virus de la famille des herpès virus selon la revendication 1, **caractérisé en ce qu'**il contient de 0,1 à 10 % en poids de piroxicam.

3. Produit pour une utilisation lors du traitement prophylactique et thérapeutique d'infections virales par un virus de la famille des herpès virus selon la revendication 2, **caractérisé en ce qu'**il contient de 1 à 5 % en poids de piroxicam.

4. Produit pour une utilisation lors du traitement prophylactique et thérapeutique d'infections virales par un virus de la famille des herpès virus selon l'une des revendications précédentes, **caractérisé en ce que** l'infection virale est une infection par herpès simplex, en particulier herpès labialis.

5. Produit pour une utilisation lors du traitement prophylactique et thérapeutique d'infections virales par un virus de la famille des herpès virus selon l'une des revendications précédentes, pour administration topique.

6. Produit pour une utilisation lors du traitement prophylactique et thérapeutique d'infections virales par un virus de la famille des herpès virus selon la revendication 5, sous forme d'une crème, d'une pommade, d'une teinture ou d'un gel.

7. Produit pour une utilisation lors du traitement prophylactique et thérapeutique d'infections virales par un virus de la famille des herpès virus selon l'une des revendications 1 à 4, pour perfusion ou injection.
